# EUROPÄISCHE PATENTSCHRIFT

(11) **EP 1 805 162 B1**
(45) Veröffentlichungstag und Bekanntmachung des Hinweises auf die Patenterteilung: **23.01.2008**
(21) Anmeldenummer: 05798084.9
(22) Anmeldetag: 20.10.2005
(51) Int. Cl.: C07D 323/06

(54) **VERFAHREN ZUR HERSTELLUNG VON TRIOXAN DADURCH GEKENNZEICHNET, DASS BEI DER ERSTEN DESTILLATIONSTUFE EIN WASSERHALTIGER SEITENSTROM ABGEZOGEN WIRD**
TRIOXANE PRODUCTION METHOD WHEREIN A SIDE AQUEOUS FLOW IS DEDUCTED AT A FIRST DISTILLATION STAGE
PROCEDE DE FABRICATION DE TRIOXANE CARACTERISE EN CE QU'UN COURANT SECONDAIRE AQUEUX EST SOUTIRE A LA PREMIERE ETAPE DE DISTILLATION

(30) Priorität: 20.10.2004 DE 102004051118
(43) Veröffentlichungstag der Anmeldung: 11.07.2007
(73) Patentinhaber: BASF AKTIENGESELLSCHAFT, 67056 Ludwigshafen (DE)
(72) Erfinder: SIEGERT, Markus, 69126 Heidelberg (DE); LANG, Neven, 68163 Mannheim (DE); STROEFER, Eckhard, 68163 Mannheim (DE); STAMMER, Achim, 67251 Freinsheim (DE); FRIESE, Thorsten, 68163 Mannheim (DE)
(74) Vertreter: Isenbruck, Günter
(86) Internationale Anmeldenummer: PCT/EP2005/011303
(87) Internationale Veröffentlichungsnummer: WO 2006/042759

(56) Entgegenhaltungen:
- EP-A- 0 133 669
- EP-A- 1 000 942
- WO-A-00/17188
- WO-A-20/04054998
- WO-A-20/05063733
- DE-A1- 19 526 307
- US-A- 5 061 349

## Beschreibung

Die Erfindung betrifft ein Verfahren zur Herstellung von Trioxan aus einer hochkonzentrierten wässrigen Formaldehydlösung.

Trioxan wird in der Regel durch Reaktivdestillation von wässriger Formaldehydlösung in Gegenwart saurer Katalysatoren hergestellt. Aus dem neben Trioxan Formaldehyd und Wasser enthaltenden Destillat wird anschließend das Trioxan mit halogenierten Kohlenwasserstoffen, wie Methylenchlorid oder 1,2-Dichlorethan, oder anderen, mit Wasser nicht mischbaren Lösungsmitteln, extrahiert.

Die DE-A 1 668 867 beschreibt ein Verfahren zur Abtrennung von Trioxan aus Wasser, Formaldehyd und Trioxan enthaltenden Gemischen durch Extraktion mit einem organischen Lösungsmittel. Dabei wird eine aus zwei Teilstrecken bestehende Extraktionsstrecke an einem Ende mit einem üblichen organischen, mit Wasser praktisch nicht mischbaren Extraktionsmittel für Trioxan beschickt, am anderen Ende mit Wasser. Zwischen den beiden Teilstrecken wird das zu trennende Destillat der Trioxan-Synthese zuführt. Auf der Seite der Lösungsmittelzuführung wird dann eine wässrige Formaldehydlösung und auf der Seite der Wasserzuführung eine praktisch formaldehydfreie Lösung von Trioxan in dem Lösungsmittel erhalten. In einem Beispiel wird das bei der Trioxan-Synthese entstandene Destillat aus 40 Gew.-% Wasser, 35 Gew.-% Trioxan und 25 Gew.-% Formaldehyd in den Mittelteil einer Pulsationskolonne eindosiert, am oberen Kolonnenende Methylenchlorid und am unteren Kolonnenende Wasser zugeführt. Dabei wird am unteren Kolonnenende eine etwa 25 gew.-%ige Lösung von Trioxan in Methylenchlorid und am oberen Kolonnenende eine etwa 30 gew.-%ige wässrige Formaldehydlösung erhalten.

Nachteil dieser Verfahrensweise ist der Anfall an Extraktionsmittel, welches aufgereinigt werden muss. Bei den verwendeten Extraktionsmitteln handelt es sich zum Teil um Gefahrenstoffe (T oder T⁺-Stoffe im Sinne der deutschen Gefahrenstoffverordnung), deren Handhabung besondere Vorsichtsmaßnahmen erfordert.

Die DE-A 197 32 291 beschreibt ein Verfahren zur Abtrennung von Trioxan aus einem wässrigen Gemisch, das im Wesentlichen aus Trioxan, Wasser und Formaldehyd besteht, bei dem man dem Gemisch Trioxan durch Pervaporation entzieht und das an Trioxan angereicherte Permeat durch Rektifikation in Trioxan und ein azeotropes Gemisch aus Trioxan, Wasser und Formaldehyd trennt. In dem Beispiel wird ein wässriges Gemisch bestehend aus 40 Gew.-% Trioxan, 40 Gew.-% Wasser und 20 Gew.-% Formaldehyd in einer ersten Destillationskolonne unter Normaldruck in ein Wasser/Formaldehyd-Gemisch und in ein azeotropes Trioxan/Wasser/Formaldehyd-Gemisch getrennt. Das azeotrope Gemisch wird in eine Pervaporationseinheit geleitet, welche eine Membran aus Polydimethylsiloxan mit einem hydrophoben Zeolithen enthält. Das mit Trioxan angereicherte Gemisch wird in einer zweiten Destillationskolonne unter Normaldruck in Trioxan und wiederum in ein azeotropes Gemisch aus Trioxan, Wasser und Formaldehyd aufgetrennt. Dieses azeotrope Gemisch wird vor die Pervaporationsstufe zurückgeführt.

Nachteilig an dieser Verfahrensweise sind die sehr hohen Investitionen für die Pervaporationseinheit.

Aus der nicht vorveröffentlichten deutschen Patentanmeldung DE 103 61 516.4 ist ein Verfahren zur destillativen Abtrennung von Trioxan aus Trioxan/Formaldehyd/Wasser-Gemischen bekannt, dass ohne Extraktions- oder Pervaporationsschritte auskommt. Das Verfahren benötigt jedoch für die Abtrennung von reinem Trioxan und reinem Waser aus dem Produktgemisch aus einem Trioxan-Synthesereaktor eine Anlage mit drei Destillationskolonnen.

Es war demgegenüber Aufgabe der Erfindung, die Wirtschaftlichkeit des Verfahrens zu verbessern, d.h. die destillative Aufarbeitung des Trioxan/Formaldehyd/Wasser-Gemischs aus der sauer katalysierten Umsetzung einer hochkonzentrierten wässrigen Formaldehydlösung mit einem geringeren Energieaufwand und höherer Ausbeute gegenüber herkömmlichen Verfahren durchzuführen.

Entsprechend wurde ein Verfahren zur Herstellung von Trioxan mit folgenden Verfahrensstufen gefunden:
- sauer katalysierte Umsetzung einer hochkonzentrierten wässrigen Formaldehydlösung in einem Reaktor unter Erhalt eines Trioxan/Formaldehyd/Wasser-Gemisches (Verfahrensstufe I),
- Destillation des Trioxan/Formaldehyd/Wasser-Gemisches aus Verfahrensstufe I unter Erhalt von Roh-Trioxan als Kopfstrom (Verfahrensstufe II) und destillative Aufarbeitung des Roh-Trioxans aus Verfahrensstufe II in einer oder mehreren weiteren Verfahrensstufen unter Erhalt von Rein-Trioxan,
das dadurch gekennzeichnet ist, dass in Verfahrensstufe II ein wasserhaltiger Seitenstrom abgezogen wird.

Es wurde gefunden, dass durch Abziehen eines wasserhaltigen Seitenstromes aus der Destillationskolonne, in der das Trioxan/Formaldehyd/Wasser-Gemisch aus der sauer katalysierten Umsetzung destillativ aufgetrennt wird, eine Betriebsweise der Destillationskolonne möglich ist, bei der als Kopfstrom ein Roh-Trioxan abgezogen wird, das annähernd die Zusammensetzung des ternären Azeotrops Trioxan/Formaldehyd/Wasser beim Kopfdruck der Kolonne aufweist und das die wirtschaftlichste Ausgangsbasis für die weitere destillative Aufarbeitung ist.

Die Verfahrensstufe I, die Umsetzung einer hochkonzentrierten wässrigen Formaldehydlösung in einem Reaktor unter Erhalt eines Trioxan/Formaldehyd/Wasser-Gemisches wird in bekannter Weise, d.h. unter homogener oder heterogener saurer Katalyse, durchgeführt. Als Katalysatoren werden häufig Schwefelsäure, Methansulfonsäure, p-Toluolsulfonsäure im Allgemeinen in einer Konzentration von 5 bis 15 Gew.-%, bezogen auf das Gesamtgewicht der wässrigen Formaldehydlösung oder eine äquivalente Menge von Ionenaustauscherharzen oder Zeolithen, eingesetzt.

Dabei wird vorliegend als hochkonzentriert eine wässrige Formaldehydlösung bezeichnet, die mindestens 55 Gew.-% Formaldehyd, oder mindestens 65 Gew.-% Formaldehyd oder auch 75 Gew.-% Formaldehyd, enthält.

Vorliegend wird als Roh-Trioxan ein Strom bezeichnet, der Trioxan in einem Gewichtsanteil von 60 bis 80 %, und daneben 30 bis 20 Gew.-% Formaldehyd sowie 10 bis 30 Gew.-% Wasser enthält.

Als Rein-Trioxan wird ein Strom bezeichnet, der mindestens 97,5 Gew.-%, bevorzugt mindestens 99 Gew.-% oder 99,9 Gew.-% oder auch 99,99 Gew.-% Trioxan enthält. An polymerisationsfähiges Rein-Trioxan werden darüber hinaus, je nach Einsatzzweck, Spezifikationen bezüglich des Ameisensäuregehaltes (häufig unter 2 Gew.-ppm) sowie des Wassergehaltes (häufig unter 50 Gew.-ppm) vorgegeben.

Als reines Wasser wird vorliegend ein Strom bezeichnet, der mindestens 95 Gew.-% oder mindestens 97,5 Gew.-%, oder auch mindestens 99 Gew.-% Wasser enthält.

Der Trioxan-Synthesereaktor ist insbesondere ein Festbett- oder Fließbettreaktor, der bei überatmosphärischem Druck betrieben wird. Bevorzugt wird der Trioxan-Synthesereaktor bei einem Druck im Bereich von 1 bis 5 bar absolut betrieben. Insbesondere soll eine Untergrenze für den Betriebsdruck nicht unterschritten werden, die einer Temperatur entspricht, bei der im Trioxan-Synthesereaktor ein Feststoffausfall stattfinden könnte.

Um die Ausbeuteverluste durch die Bildung des Nebenproduktes Ameisensäure zu begrenzen, wird der Trioxan-Synthesereaktor bevorzugt bei Verweilzeiten unterhalb von 30 Minuten, besonders bevorzugt unterhalb von 15 Minuten, betrieben.

Hierzu wird insbesondere die hochkonzentrierte wässrige Formaldehydlösung dem Trioxan-Synthesereaktor über einen Zwangsumlaufentspannungsverdampfer zugeführt.

Aus dem Trioxan-Synthesereaktor wird aus dem oberen Bereich desselben ein gasförmiger Strom, enthaltend Trioxan, Formaldehyd und Wasser, abgezogen. Die Zusammensetzung des gasförmigen Stromes aus dem Trioxan-Synthesereaktor entspricht im Allgemeinen 1 bis 25 Gew.-% Trioxan, 50 bis 80 Gew.-% Formaldehyd und 10 bis 25 Gew.-% Wasser.

Der gasförmige Strom aus dem Trioxan-Synthesereaktor wird, vorzugsweise über ein Regelventil, in die nachgeschaltete Kolonne entspannt, in der in Verfahrensstufe II das Trioxan/Formaldehyd/Wasser-Gemisch in einen Kopfstrom, enthaltend Roh-Trioxan und einen Sumpfstrom aufgetrennt wird, der bevorzugt in den Trioxan-Synthesereaktor recycliert wird.

Dieser Umlaufstrom wird mit der frisch zugeführten hochkonzentrierten Formaldehydlösung entweder vor der Umlaufpumpe oder in einem Reaktionsmischer vermengt. Besonders vorteilhaft kann für diese Funktion ein statischer Mischer eingesetzt werden. Alternativ ist es auch möglich, den Umlaufstrom und den frischen wässrigen Formaldehydstrom jeweils getrennt und nur getaucht in den Trioxan-Synthesereaktor zu führen.

Der Kopfstrom, enthaltend Roh-Trioxan, wird in einer oder mehreren weiteren Verfahrensstufen destillativ zu Rein-Trioxan gereinigt.

Die Druckdifferenz zwischen der Kolonne, in der die Verfahrensstufe II durchgeführt wird und dem Trioxan-Synthesereaktor kann alternativ zum Regelventil beispielsweise durch hydrostatischen Druck ausgeglichen werden.

Da das Trioxan-haltige Reaktionsgemisch aus dem Trioxan-Synthesereaktor gasförmig abgezogen wird, verbleiben die für den Feststoffausfall von Para-Formaldehyd kritischen Säuren im Trioxan-Synthesereaktor und werden nicht in die Destillationskolonne mitgeschleppt. Dadurch können für die Kolonne kostengünstigere Stahlsorten eingesetzt werden, die nicht säurebeständig sein müssen.

Die Zusammensetzung des als Kopfstrom in Verfahrensstufe II abgezogenen Rohtrioxans wird so vorgegeben, dass sie der Zusammensetzung des ternären Azeotrops Trioxan/Formaldehyd/Wasser beim Kopfdruck der Kolonne entspricht, weil dies die wirtschaftlichste Ausgangsbasis für die weitere destillative Aufarbeitung ist.

Erfindungsgemäß wird in Verfahrensstufe II ein wasserhaltiger Seitenstrom abgezogen, vorzugsweise in flüssiger Form.

Der wasserhaltige Seitenstrom enthält vorteilhaft 10 bis 90 Gew.-%, bevorzugt 40 bis 80 Gew.-%, besonders bevorzugt 50 bis 80 Gew.-%, Wasser.

Die Verfahrensstufe II wird vorteilhaft in einer Kolonne mit 5 bis 40 theoretischen Trennstufen durchgeführt, die bei einem Kopfdruck zwischen 0,05 und 2,50 bar absolut betrieben wird.

Weiter bevorzugt wird die Kolonne, in der die Verfahrensstufe II durchgeführt wird, mit 5 bis 20 theoretischen Trennstufen ausgelegt und bei einem Kopfdruck zwischen 0,20 und 0,75 bar absolut betrieben.

Die Positionierung des Abzugs für den wässrigen Seitenstrom in Verfahrensstufe II wird vorteilhaft auf eine theoretische Trennstufe gelegt, deren Position zwischen 10% und 90% der Gesamtzahl der theoretischen Trennstufen in der Kolonne liegt.

Vorteilhaft sind der Reaktor, in der die Verfahrensstufe I durchgeführt wird und die Kolonne, in der die Verfahrensstufe II durchgeführt wird, zu einer Einheit verschaltet, dergestalt, dass der aus dem Reaktor aufsteigende Brüden unmittelbar in die Kolonne und die aus der Kolonne ablaufende Flüssigkeit unmittelbar in den Reaktor eintritt.

Um das Aufpegeln von Schwersiedern, vorliegend insbesondere Dimethoxydimethylether und Ameisensäure, zu verhindern, wird vorteilhaft aus dem Reaktor, in dem die Verfahrensstufe I durchgeführt wird, oder aus der Kolonne, in der die Verfahrensstufe II durchgeführt, kontinuierlich oder absatzweise ein Strom von 0,01 bis 1 Gew.-% der Zulaufmenge in den Reaktor, insbesondere von 0,1 bis 1,0 Gew.-% der Zulaufmenge in den Reaktor, ausgeschleust.

Die weitere destillative Aufarbeitung des in Verfahrensstufe I abgezogenen Rohtrioxans kann bevorzugt in der Weise erfolgen, dass das Rohtrioxan einer Kolonne zugeführt wird, in der ein Leichtsieder enthaltender Strom abgetrennt wird, wobei die Kolonne vorteilhaft 5 bis 50 theoretische Trennstufen umfasst und bei einem Kopfdruck zwischen 0,1 und 5 bar absolut betrieben wird. Weiter bevorzugt wird die Kolonne zur Abtrennung der Leichtsieder mit 10 bis 30 theoretischen Trennstufen ausgelegt und bei einem Kopfdruck zwischen 1,0 und 2,5 bar absolut betrieben.

Als Leichtsieder werden vorliegend Substanzen verstanden, deren Siedepunkt unterhalb des Siedepunkts von reinem Trioxan liegt; es sind dies insbesondere Methylal, Methanol und Methylformiat.

Die Kolonne, in der die Leichtsieder abgetrennt werden, wird bevorzugt in der Weise ausgelegt, dass der Verstärkungsteil derselben 25 bis 95%, bevorzugt 50 bis 75% der Gesamtzahl der theoretischen Trennstufen der Kolonne aufweist.

Der Sumpfstrom aus der Kolonne, aus der die Leichtsieder abgezogen werden, wird einer Trioxan-Reinkolonne zugeführt, in der Reintrioxan als Seitenabzug oder als Sumpfstrom gewonnen wird. Die Trioxan-Reinkolonne wird bevorzugt bei einem Kopfdruck betrieben, der 0,10 bis 10,0 bar höher liegt als der Kopfdruck der Kolonne, in der die Verfahrensstufe II durchgeführt wird.

Bevorzugt wird der Kopfstrom aus der Trioxan-Reinkolonne einer weiteren Kolonne zugeführt, in der als Sumpfstrom reines Wasser abgezogen wird. Diese Kolonne ist bevorzugt mit 5 bis 50, insbesondere mit 10 bis 30 theoretischen Trennstufen ausgelegt und wird bei einem Kopfdruck zwischen 1,0 und 10 bar absolut, bevorzugt bei einem Kopfdruck zwischen 2,5 und 6,5 bar absolut betrieben.

Die Trioxan-Reinkolonne und/oder die Kolonne, in der als Sumpfstrom reines Wasser abgezogen wird, wird bevorzugt in der Weise ausgelegt, dass der Abtriebsteil 25 bis 100%, bevorzugt 75 bis 100%, besonders bevorzugt 90 bis 100% der Gesamtzahl der theoretischen Trennstufen der Kolonne aufweist.

Vorteilhaft wird der Seitenstrom aus der Kolonne, in der die Verfahrensstufe II durchgeführt wird, und/oder ein weiterer wasserhaltiger Strom der Kolonne, in der reines Wasser gewonnen wird, zugeführt und/oder der Kopfstrom aus der Kolonne, in der reines Wasser gewonnen wird, der Kolonne zugeführt, in der die Verfahrensstufe II durchgeführt wird.

Der weitere wasserhaltige Strom, der der Kolonne zugeführt wird, in der reines Trioxan gewonnen wird, enthält vorzugsweise keine verfahrensfremden Komponenten und hat vorzugsweise einen Wassergehalt von mindestens 10 Gew.-%, insbesondere von mindestens 50 Gew.-%.

Vorteilhaft kann anstelle der Trioxan-Reinkolonne und der Kolonne, in der reines Wasser gewonnen wird, eine Trennwandkolonne eingesetzt werden, in der ein Sumpfstrom, enthaltend Rein-Trioxan und ein Seitenstrom, enthaltend reines Wasser, abgezogen werden.

Die Erfindung wird im Folgenden anhand einer Zeichnung näher erläutert.

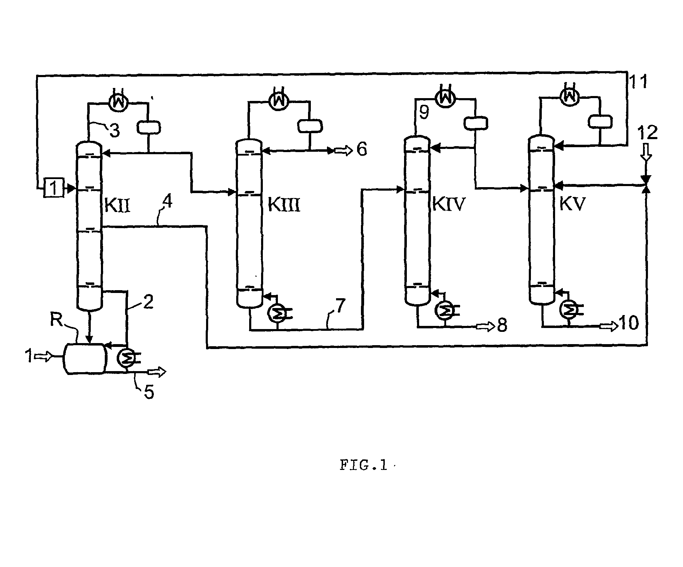

Figur 1 zeigt die schematische Darstellung einer bevorzugten Anlage nach der Erfindung. Eine hochkonzentrierte wässrige Formaldehydlösung, Strom 1, wird einem Reaktor R unter Erhalt eines Trioxan/Formaldehyd/Wasser-Gemisches, Strom 2, zugeführt. Strom 2 wird in einer Kolonne K II in einen Roh-Trioxan-Kopfstrom 3 und einen wasserhaltigen Seitenstrom 4 aufgetrennt.

Der Kopfstrom 3 wird teilweise als Rücklauf wieder auf die Kolonne K II aufgegeben und im Übrigen einer Kolonne K III zugeführt, in der über Kopf Leichtsieder, Strom 6, abgetrennt werden. Der Sumpfstrom 7 aus der Kolonne K III wird einer Trioxan-Reinkolonne K IV zugeführt, in der in der Figur dargestellten bevorzugten Ausführungsform Rein-Trioxan als Sumpfstrom 8 abgezogen wird. Der Kopfstrom 9 aus der Kolonne K IV wird einer weiteren Kolonne K V zugeführt, in der als Sumpfstrom 10 reines Wasser abgetrennt wird sowie ein Kopfstrom 11, der in der in Figur dargestellten bevorzugten Ausführungsform in die Kolonne K II recycliert wird. Der Kolonne K V wird der wasserhaltige Seitenstrom 4 aus der Kolonne K II sowie ein weiterer wasserhaltiger Seitenstrom 12 zugeführt.

## Patentansprüche

1. Verfahren zur Herstellung von Trioxan mit folgenden Verfahrensstufen:
- sauer katalysierte Umsetzung einer hochkonzentrierten wässrigen Formaldehydlösung (1) in einem Reaktor (R) unter Erhalt eines Trioxan/Formaldehyd/Wasser-Gemisches (2) (Verfahrensstufe I),
- Destillation des Trioxan/Formaldehyd/Wasser-Gemisches (2) aus Verfahrensstufe I unter Erhalt von Roh-Trioxan als Kopfstrom (3) (Verfahrensstufe II) und
- destillative Aufarbeitung des Roh-Trioxans (3) aus Verfahrensstufe II in einer oder mehreren weiteren Verfahrensstufen unter Erhalt von Rein-Trioxan (8),
**dadurch gekennzeichnet, dass** in Verfahrensstufe II ein wasserhaltiger Seitenstrom (4) abgezogen wird.

2. Verfahren nach Anspruch 1, **dadurch gekennzeichnet, dass** der in Verfahrensstufe II abgezogene Seitenstrom (4) flüssig ist.

3. Verfahren nach Anspruch 1 oder 2, **dadurch gekennzeichnet, dass** der in Verfahrensstufe II abgezogene wasserhaltige Seitenstrom (4) 10 bis 90 Gew.-%, bevorzugt 40 bis 80 Gew.-%, besonders bevorzugt 50 bis 80 Gew.-% Wasser enthält.

4. Verfahren nach einem der Ansprüche 1 bis 3, **dadurch gekennzeichnet, dass** die Verfahrensstufe II in einer Kolonne K II mit 5 bis 40 theoretischen Trennstufen durchgeführt wird, die bei einem Kopfdruck zwischen 0,05 und 2,50 bar absolut betrieben wird.

5. Verfahren nach Anspruch 4, **dadurch gekennzeichnet, dass** die Kolonne K II 5 bis 20 theoretische Trennstufen aufweist und bei einem Kopfdruck zwischen 0,20 und 0,95 bar absolut betrieben wird.

6. Verfahren nach einem der Ansprüche 1 bis 5, **dadurch gekennzeichnet, dass** der Seitenstrom (4) aus der Kolonne K II von einer theoretischen Trennstufe abgezogen wird, deren Position zwischen 10% und 90% der Gesamtzahl der theoretischen Trennstufen in der Kolonne liegt.

7. Verfahren nach einem der Ansprüche 1 bis 6, **dadurch gekennzeichnet, dass** der Reaktor (R), in der die Verfahrensstufe I durchgeführt wird und die Kolonne K II eine Einheit bilden, dergestalt, dass der aus dem Reaktor aufsteigende Brüden unmittelbar in die Kolonne K II und die aus der Kolonne K II ablaufende Flüssigkeit unmittelbar in den Reaktor (R) eintritt.

8. Verfahren nach einem der Ansprüche 1 bis 7, **dadurch gekennzeichnet, dass** aus dem Reaktor (R), in dem die Verfahrensstufe I durchgeführt wird oder aus der Kolonne K II kontinuierlich oder absatzweise ein Strom (5) von 0,01 bis 1 Gew.-% der Zulaufmenge in den Reaktor (R), bevorzugt von 0,1 bis 1,0 Gew.-% der Zulaufmenge in den Reaktor (R), ausgeschleust wird.

9. Verfahren nach einem der Ansprüche 1 bis 8, **dadurch gekennzeichnet, dass** der Kopfstrom (3) aus der Kolonne K II einer Kolonne K III zugeführt wird, in der über Kopf ein Leichtsieder enthaltender Strom (6) abgetrennt wird, wobei die Kolonne K III 5 bis 50 theoretische Trennstufen umfasst und bei einem Kopfdruck zwischen 0,1 und 5,0 bar absolut betrieben wird.

10. Verfahren nach Anspruch 9, **dadurch gekennzeichnet, dass** die Kolonne K III 10 bis 30 theoretische Trennstufen aufweist und bei einem Kopfdruck zwischen 1,0 und 2,5 bar absolut betrieben wird.

11. Verfahren nach Anspruch 9 oder 10, **dadurch gekennzeichnet, dass** der Verstärkungsteil der Kolonne K III 25 bis 95%, bevorzugt 50 bis 75%, der Gesamtzahl der theoretischen Trennstufen der Kolonne K III aufweist.

12. Verfahren nach einem der Ansprüche 1 bis 11, **dadurch gekennzeichnet, dass** der Sumpfstrom (7) aus der Kolonne K III einer Trioxan-Reinkolonne K IV zugeführt wird, in der Rein-Trioxan als Seitenabzug oder Sumpfstrom (8) gewonnen wird, und die bei einem Kopfdruck betrieben wird, der um 0,10 bis 10,0 bar höher liegt als der Kopfdruck der Kolonne K II.

13. Verfahren nach Anspruch 12, **dadurch gekennzeichnet, dass** der Kopfstrom (9) aus der Trioxan-Reinkolonne K IV einer Kolonne K V zugeführt wird, in der als Sumpfstrom (10) reines Wasser abgezogen wird und die 5 bis 50 theoretische Trennstufen aufweist und bei einem Kopfdruck zwischen 1,0 und 10,0 bar absolut betrieben wird.

14. Verfahren nach Anspruch 13, **dadurch gekennzeichnet, dass** die Kolonne K V 10 bis 30 theoretische Trennstufen aufweist und bei einem Kopfdruck zwischen 2,5 und 6,5 bar absolut betrieben wird.

15. Verfahren nach einem der Ansprüche 12 bis 14, **dadurch gekennzeichnet, dass** der Abtriebsteil der Kolonne K IV und/oder der Kolonne K V 25 bis 100%, bevorzugt 75 bis 100%, besonders bevorzugt 90 bis 100% der Gesamtzahl der theoretischen Trennstufen der Kolonne aufweist.

16. Verfahren nach einem der Ansprüche 13 bis 15, **dadurch gekennzeichnet, dass** der Seitenstrom (4) aus der Kolonne K II der Kolonne K V und/oder der Kopfstrom (11) aus der Kolonne K V der Kolonne K II und/oder ein weiterer wasserhaltiger Strom (12) der Kolonne K V zugeführt wird.

17. Verfahren nach einem der Ansprüche 12 bis 16, **dadurch gekennzeichnet, dass** anstelle der Kolonnen K IV und K V eine Trennwandkolonne eingesetzt wird, aus der ein Sumpfstrom, enthaltend Rein-Trioxan und ein Seitenstrom, enthaltend reines Wasser, abgezogen werden.

## Claims

1. A process for preparing trioxane, comprising the following process stages:
- acid-catalyzed reaction of a highly concentrated aqueous formaldehyde solution (1) in a reactor (R) to obtain a trioxane/formaldehyde/water mixture (2) (process stage I),
- distillation of the trioxane/formaldehyde/water mixture (2) from process stage I to obtain crude trioxane as the top stream (3) (process stage II) and
- distillative workup of the crude trioxane (3) from process stage II in one or more further process stages to obtain pure trioxane (8),
which comprises drawing off an aqueous side stream (4) in process stage II.

2. The process according to claim 1, wherein the side stream (4) drawn off in process stage II is liquid.

3. The process according to claim 1 or 2, wherein the aqueous side stream (4) drawn off in process stage II comprises from 10 to 90% by weight, preferably from 40 to 80% by weight, more preferably from 50 to 80% by weight, of water.

4. The process according to any of claims 1 to 3, wherein process stage II is operated in a column K II which has from 5 to 40 theoretical plates and is operated at a top pressure between 0.05 and 2.50 bar absolute.

5. The process according to claim 4, wherein the column K II has from 5 to 20 theoretical plates and is operated at a top pressure between 0.20 and 0.95 bar absolute.

6. The process according to any of claims 1 to 5, wherein the side stream (4) from the column K II is drawn off from a theoretical plate whose position is between 10% and 90% of the total number of theoretical plates in the column.

7. The process according to any of claims 1 to 6, wherein the reactor (R) in which process stage I is carried out and the column K II form one unit, in such a way that the vapors rising out of the reactor directly enter the column K II and the liquid effluxing out of the column K II directly enters the reactor (R).

8. The process according to any of claims 1 to 7, wherein a stream (5) of from 0.01 to 1% by weight of the feed amount into the reactor (R), preferably of from 0.1 to 1.0% by weight of the feed amount into the reactor (R), is discharged continuously or batchwise from the reactor (R) in which process stage I is carried out or from the column K II.

9. The process according to any of claims 1 to 8, wherein the top stream (3) from the column K II is fed to a column K III in which a stream (6) comprising low boilers is removed overhead, the column K III comprising from 5 to 50 theoretical plates and being operated at a top pressure between 0.1 and 5.0 bar absolute.

10. The process according to claim 9, wherein the column K III has from 10 to 30 theoretical plates and is operated at a top pressure between 1.0 and 2.5 bar absolute.

11. The process according to claim 9 or 10, wherein the rectifying section of the column K III has from 25 to 95%, preferably from 50 to 75%, of the total number of theoretical plates of the column K III.

12. The process according to any of claims 1 to 11, wherein the bottom stream (7) from the column K III is fed to a trioxane purifying column K IV in which pure trioxane is obtained as a side draw or bottom stream (8), and which is operated at a top pressure which is from 0.10 to 10.0 bar higher than the top pressure of the column K II.

13. The process according to claim 12, wherein the top stream (9) from the trioxane purifying column K IV is fed to a column K V in which the bottom stream (10) drawn off is pure water, and which has from 5 to 50 theoretical plates and is operated at a top pressure between 1.0 and 10.0 bar absolute.

14. The process according to claim 13, wherein the column K V has from 10 to 30 theoretical plates and is operated at a top pressure between 2.5 and 6.5 bar absolute.

15. The process according to any of claims 12 to 14, wherein the stripping section of the column K IV and/or of the column K V has from 25 to 100%, preferably from 75 to 100%, more preferably from 90 to 100%, of the total number of theoretical plates of the column.

16. The process according to any of claims 13 to 15, wherein the side stream (4) from the column K II is fed to the column K V and/or the top stream (11) from the column K V is fed to the column K II and/or a further aqueous stream (12) is fed to the column K V.

17. The process according to any of claims 12 to 16, wherein, instead of the columns K IV and K V, a dividing wall column is used from which a bottom stream comprising pure trioxane and a side stream comprising pure water are drawn off.

## Revendications

1. Procédé pour la préparation de trioxane avec les étapes de procédé suivantes :
- réaction à catalyse acide d'une solution de formaldéhyde aqueuse à haute concentration (1) dans un réacteur (R) avec obtention d'un mélange trioxane/formaldéhyde/eau (2) (étape de procédé I),
- distillation du mélange trioxane/formaldéhyde/eau (2) à partir de l'étape de procédé I avec obtention de trioxane brut en tant que courant de tête (3) (étape de procédé II) et
- traitement par distillation du trioxane brut (3) à partir de l'étape de procédé II en une ou plusieurs autres étapes de procédé avec obtention de trioxane pur (8),
**caractérisé en ce que** dans l'étape de procédé II, un courant latéral contenant de l'eau (4) est extrait.

2. Procédé suivant la revendication 1, **caractérisé en ce que** le courant latéral extrait dans l'étape de procédé II (4) est liquide.

3. Procédé suivant la revendication 1 ou 2, **caractérisé en ce que** le courant latéral (4) contenant de l'eau extrait dans l'étape de procédé II contient 10 à 90 % en poids, de préférence 40 à 80 % en poids et, mieux encore, 50 à 80 % en poids d'eau.

4. Procédé suivant l'une des revendications 1 à 3, **caractérisé en ce que** l'étape de procédé II est exécutée dans une colonne K II avec 5 à 40 étages de séparation théoriques, qui est exploitée pour une pression de tête comprise entre 0,05 et 2,50 bars absolus.

5. Procédé suivant la revendication 4, **caractérisé en ce que** la colonne K II présente 5 à 20 étages de séparation théoriques et est exploitée pour une pression de tête comprise entre 0,20 et 0,95 bar absolu.

6. Procédé suivant l'une des revendications 1 à 5, **caractérisé en ce que** le courant latéral (4) est extrait de la colonne K II d'un étage de séparation théorique dont la position dans la colonne se situe entre 10 et 90 % du nombre total d'étages de séparation théoriques.

7. Procédé suivant l'une des revendications 1 à 6, **caractérisé en ce que** le réacteur (R) dans lequel l'étape de procédé I est exécutée et la colonne K II forment une unité de telle sorte que la vapeur montant du réacteur pénètre directement dans la colonne K II et que le liquide s'écoulant de la colonne K II pénètre directement dans le réacteur (R).

8. Procédé suivant l'une des revendications 1 à 7, **caractérisé en ce qu'**à partir du réacteur (R) dans lequel l'étape de procédé I est exécutée ou à partir de la colonne K II, un courant (5) de 0,01 à 1 % en poids de la quantité d'introduction est amené de manière continue ou discontinue dans le réacteur (R), de préférence une quantité de 0,1 à 1,0 % en poids de la quantité d'introduction dans le réacteur (R).

9. Procédé suivant l'une des revendications 1 à 8, **caractérisé en ce que** le courant de tête (3) est amené de la colonne K II à une colonne K III, dans laquelle un courant (6) contenant un corps à bas point d'ébullition est séparé par la tête, la colonne K III comprenant 5 à 50 étages de séparation théoriques et est exploitée pour une pression de tête comprise entre 0,1 et 5,0 bars absolus.

10. Procédé suivant la revendication 9, **caractérisé en ce que** la colonne K III présente 10 à 30 étages de séparation théoriques et est exploitée pour une pression de tête comprise entre 1,0 et 2,5 bars absolus.

11. Procédé suivant la revendication 9 ou 10, **caractérisé en ce que** la partie de renforcement de la colonne K III présente 25 à 95 %, de préférence 50 à 75 % du nombre total des étages de séparation théoriques de la colonne K III.

12. Procédé suivant l'une des revendications 1 à 11, **caractérisé en ce que** le courant de fond (7) provenant de la colonne K III est amené à une colonne de trioxane pur K IV dans laquelle du trioxane pur est extrait en tant qu'extraction latérale ou courant de fond (8) et qui est exploitée pour une pression de tête qui est de 0,10 à 10,0 bars supérieure à la pression de tête de la colonne K II.

13. Procédé suivant la revendication 12, **caractérisé en ce que** le courant de tête (9) est amené à partir de la colonne de trioxane pur K IV à une colonne K V, dans laquelle, comme courant de fond (10), de l'eau pure est extraite et qui présente 5 à 50 étages de séparation théoriques et est exploitée pour une pression de tête comprise entre 1,0 et 10,0 bars absolus.

14. Procédé suivant la revendication 13, **caractérisé en ce que** la colonne K V présente 10 à 30 étages de séparation théoriques et est exploitée pour une pression de tête comprise entre 2,5 et 6,5 bars absolus.

15. Procédé suivant l'une des revendications 12 à 14, **caractérisé en ce que** la partie extraction de la colonne K IV et/ou de la colonne K V présente 25 à 100 %, de préférence 75 à 100 %, mieux encore 90 à 100 % du nombre total d'étages de séparation théoriques de la colonne.

16. Procédé suivant l'une des revendications 13 à 15, **caractérisé en ce que** le courant latéral (4) provenant de la colonne K II est conduit à la colonne K V et/ou que le courant de tête (11) provenant de la colonne K V est conduit à la colonne K II et/ou un autre courant contenant de l'eau (12) est conduit à la colonne K V.

17. Procédé suivant l'une des revendications 12 à 16, **caractérisé en ce que**, en lieu et place des colonnes K IV et K V, on met en oeuvre une colonne à paroi de séparation à partir de laquelle un courant de fond, contenant du trioxane pur et un courant latéral, contenant de l'eau pure, sont extraits.
